# EUROPEAN PATENT APPLICATION

(11) **EP 4 682 263 A1**
(43) Date of publication of application: **21.01.2026**
(21) Application number: 24934357.5
(22) Date of filing: 17.06.2024
(51) Int. Cl.: C12P 21/06

(54) **PREPARATION METHOD FOR TELOPEPTIDE-RETAINED COLLAGEN AND TELOPEPTIDE-RETAINED COLLAGEN**

(30) Priority: 29.05.2024 CN 202410688864
(71) Applicant: Huizhou Foryou Medical Devices Co., Ltd., Huizhou, Guangdong 516001 (CN)
(72) Inventor: DENG, Tianfa, Huizhou, Guangdong 516001 (CN); ZHOU, Zhaotong, Huizhou, Guangdong 516001 (CN)
(74) Representative: Herrero & Asociados, S.L.
(86) International application number: PCT/CN2024/099639
(87) International publication number: WO 2025/245931

(57) **Abstract**

A method for preparing a telopeptide-retained collagen includes: preparing a collagen suspension; treating the collagen suspension with a protease to remove non-collagen protein impurities, so as to obtain a collagen extract; dissolving and homogenizing the collagen extract to obtain the telopeptide-retained collagen. The protease has a higher enzymatic efficiency for the non-collagen protein impurities than for a telopeptide of a collagen.

## Description

The present application claims the priority of the Chinese patent application No. 202410688864.0, filed on May 29, 2024, in the title of "METHOD FOR PREPARING TELOPEPTIDE-RETAINED COLLAGEN AND TELOPEPTIDE-RETAINED COLLAGEN", contents of which are incorporated herein by its entireties.

### TECHNICAL FIELD

Embodiments of the present disclosure relate to the technical field of preparing collagens, and more specifically, to a method for preparing a telopeptide-retained collagen and a telopeptide-retained collagen.

### BACKGROUND

Collagen of animal origins has been widely used in medical and health care. In an early stage of application, it has been believed that the collagen has almost no immunogenicity due to amino acid sequences of the collagen being highly conserved. However, as research and application continue, the collagen exhibits immunogenicity. A telopeptide region of the collagen, compared to a helical domain, exhibits higher immunogenicity. Therefore, most commercial collagens, especially soluble collagens, have the telopeptide removed, so as to reduce the immunogenicity. In addition, most collagens reported in domestic patents are also prepared by removing the telopeptide. However, from the perspective of material sciences, retention of the telopeptide retention is significant, a more complete structure and more cross-linking sites of a material are maintained, more possible downstream applications may be available for the collagen. According to studies, the telopeptide is significant in fiber formation, thermal stability, triple helix structure stability, mechanical performance and cell recognition and adhesion. Therefore, how to prepare a collagen retaining the telopeptide (retaining the telopeptide at an N-terminal and at a C-terminal thereof) has become an urgent problem to be solved.

### SUMMARY

The present disclosure provides a method for preparing a telopeptide-retained collagen and a telopeptide-retained collagen, so as to obtain collagens having a higher retention rate of telopeptides.

In a first aspect, the present disclosure provides a method for preparing a telopeptide-retained collagen, including: preparing a collagen suspension; treating the collagen suspension with a protease to remove non-collagen protein impurities, so as to obtain a collagen extract, wherein the protease has a higher enzymatic efficiency for the non-collagen protein impurities than for a telopeptide of a collagen; dissolving and homogenizing the collagen extract to obtain the telopeptide-retained collagen.

In some embodiments, the treating the collagen suspension with a protease to remove non-collagen protein impurities, so as to obtain a collagen extract, includes: adding a neutral protease and/or an alkaline protease to the collagen suspension to obtain a first mixture, adjusting a pH of the first mixture to an optimal pH range in which the neutral protease and/or the alkaline protease having optimal activities; leaving the first mixture to undergo reactions at a first predetermined temperature for a first predetermined time length to obtain a second mixture; centrifuging, filtering and water washing the second mixture to obtain the collagen extract.

In some embodiments, the protease includes the neutral protease, the optimum pH range for the neutral protease having the optimal activities is 6 to 8; and when the protease comprises the alkaline protease, the optimum pH range for the alkaline protease having the optimal activities is 7 to 10; and/or the first predetermined temperature is in a range of 4°C to 20°C; and/or, the first predetermined time length is in a range of 4h to 8h.

In some embodiments, when adding the neutral protease and/or the alkaline protease to the collagen suspension, the method further includes: adding an enzyme stabilizer to the collagen suspension.

In some embodiments, the enzyme stabilizer includes at least one of propylene glycol, polyethylene glycol, sucrose and mannitol.

In some embodiments, the protease includes one or more of *Bacillus licheniformis* alkaline protease, *Aspergillus oryzae* alkaline protease, *Bacillus subtilis* alkaline protease, *Streptomyces griseus* alkaline protease and *Fusarium* alkaline protease.

In some embodiments, before treating the collagen suspension with the protease, the method further includes: adding a collagen terminal-group protector to the collagen suspension.

In some embodiments, the collagen terminal-group protector includes at least one of highly unsaturated fatty acids and amino acids.

In some embodiments, the dissolving and homogenizing the collagen extract to obtain the telopeptide-retained collagen, includes: adding an acidic solution to the collagen extract to obtain an acidic dissolving solution; homogenizing the acidic dissolving solution under a high pressure to obtain the telopeptide-retained collagen.

In some embodiments, the acidic solution includes at least one of acetic acid, hydrochloric acid, sulfuric acid or citric acid.

In some embodiments, the dissolving and homogenizing the collagen extract to obtain the telopeptide-retained collagen, includes: freezing and drying the telopeptide-retained collagen to obtain the telopeptide-retained collagen in a dried state.

In some embodiments, the preparing a collagen suspension, includes: selecting an animal tissue comprising the collagen; refining the animal tissue into sub-tissues; treating the sub-tissues sequentially with an organic reagent, a salt solution, an acid-enzyme mixture, and an alkaline solution, so as to obtain a pretreatment product; and homogenizing and grinding the pretreatment product, so as to obtain the collagen suspension.

In some embodiments, the homogenizing and grinding the pretreatment product, so as to obtain the collagen suspension, includes: transferring the pretreatment product to a homogenizing and grinding device, adding an alkaline solution, and homogenizing and grinding the pretreatment product to have a particle size of 20 µm to 400 µm, so as to obtain the collagen suspension.

In a second aspect, the present disclosure provides a telopeptide-retained collagen, prepared from the method of the above embodiments.

In some embodiments, a telopeptide retention rate of the telopeptide-retained collagen is greater than or equal to 80%.

According to the present disclosure, a method for preparing a telopeptide-retained collagen is provided. A protease may be added to a collagen suspension for enzymatic hydrolysis. The protease has a higher enzymatic hydrolysis efficiency for non-collagen proteins than that for telopeptides of collagens. In this way, degradation of the telopeptides may be reduced while the non-collagen proteins are degraded, such that the non-collagen proteins and other impurities are removed, and the telopeptides are retained. A triple helix structure of the obtained telopeptide-retaining collagen may be complete, fewer non-collagen protein impurities are left with the obtained telopeptide-retaining collagen, a high rate of telopeptide retention may be achieved. Therefore, a wide range of application may be achieved. The method for preparing the telopeptide-retained collagen of the present disclosure have simple and easy operations, the enzymatic reaction can be controlled, a source of enzymes is achievable, and any equipment for performing conventional reaction conditions can be used. By performing the method of the present application, the telopeptide-retained collagen may be prepared in an industrialization scale, having significant economic values.

### BRIEF DESCRIPTION OF THE DRAWINGS

To more clearly illustrate technical solutions in the embodiments of the present disclosure or the related art, the accompanying drawings needed for describing the embodiments of the present disclosure or the related art will be briefly introduced in the following. Obviously, the drawings in the following description are only some embodiments of the present disclosure, and any ordinary skilled person in the art may obtain other drawings based on these drawings without creative work.
FIG. 1 is a flow chart of a method for preparing a telopeptide-retained collagen according to an embodiment of the present disclosure.
FIG. 2 is a flow chart of the method for preparing the telopeptide-retained collagen according to an embodiment of the present disclosure.
FIG. 3 is a flow chart of the method for preparing the telopeptide-retained collagen according to an embodiment of the present disclosure.
FIG. 4 is a flow chart of the method for preparing the telopeptide-retained collagen according to an embodiment of the present disclosure.
FIG. 5 is a flow chart of the method for preparing the telopeptide-retained collagen according to an embodiment of the present disclosure.
FIG. 6 is an SDS-PAGE electrophoresis gel image of collagens obtained from an embodiment 1, an embodiment 2, and an embodiment 3.
FIG. 7 is an image of circular dichroism (CD) spectrum of the collagen obtained from the embodiment 1.
FIG. 8 is an image of circular dichroism (CD) spectrum of the collagen obtained from the embodiment 2.
FIG. 9 is a differential scanning calorimetry (DSC) thermogram image of the collagen obtained from the embodiment 3.

### DETAILED DESCRIPTIONS

The technical solutions in the embodiments of the present disclosure will be clearly and completely described below by referring to the accompanying drawings in the embodiments of the present disclosure. Apparently, the described embodiments are only a part of, not all of, the embodiments of the present disclosure. All other embodiments, which are obtained by any ordinary skilled person in the art based on the embodiments in the present disclosure without making creative work, shall fall within the scope of the present disclosure.

Terms "first", "second", and "third" in the present disclosure are used for descriptive purposes only and are not to indicate or imply relative importance or implicitly specifying the number of technical features. The term "and/or" is merely description of an association relationship of associated objects, indicating that three kinds of relationships may exist. For example, A and/or B may mean that, the A exits alone, the A and the B exit at the same time, and the B exits alone. In addition, the character "/" in the present disclosure generally indicates an object before the character "or" an object after the character. In addition, "a plurality of" in the present disclosure means two or more than two.

Collagen is an important component of extracellular matrix (ECM) of vertebrates and is widely distributed in connective tissues, including skin, bones, tendons, cartilage, cornea, and teeth, and is the most abundant protein in mammals, accounting for approximately 20%-30% of total protein contents. The collagen has ideal biocompatibility, biodegradability, low immunogenicity and special biological activities. The collagen is significant in cell adhesion, cell migration, angiogenesis, tissue morphogenesis and tissue repair. The collagen may serve as a scaffolding material exhibits tissue affinity and macromolecular properties, similar to those of natural extracellular matrix. The collagen are widely used in biomedical application, including sutures, tissue replacement and regeneration, medical aesthetics, trauma repair, dental dressings and skin regeneration templates.

When the collagen is to be prepared from animal tissues, most common tissue sources of the collagen are the skin and achillea tendons. In the skin and achillea tendons, type I collagen accounts for more than 90% of all proteins, and a small amount of type III collagen may also be present. Each of the type I collagen and the type III collagen includes three parts: a non-helical amino-terminal region (N-terminal peptide), a central triple-helical region, and a non-helical carboxy-terminal region (C-terminal peptide). The triple helix structure is formed of three lefthanded polypeptide chains (α-chains) linked to each other by hydrogen bonds between peptide bonds (NH) of glycine residues of one peptide and carbonyl groups (C=O) of an adjacent polypeptide. The triple helix structure is an active region of the collagen exerting functional properties. An intact triple helix structure promotes cellular adhesion/migration, regulates/promotes cellular differentiation, and facilitates healing. Short segments of the C-terminal telopeptide and the N-terminal telopeptide (terminal peptides) are non-helical and have an uncommon amino acid, which is hydroxylysine. Due to an action of a lysyl oxidase, lysine or hydroxylysine residues in the N-terminal telopeptides and the C-terminal telopeptides of adjacent molecules may undergo a covalent aldol reaction to enable the adjacent molecules to be linked to each other along a fiber from head to tail. The action of the lysyl oxidase is significant for formation and stabilization of collagen fibers. The lysyl oxidase catalyzes the covalent aldol reaction of the lysine or the hydroxylysine residues in the N-terminal telopeptides and the C-terminal peptides of adjacent molecules to generate aldimine cross-linking substance, such that mechanical strength of collagen tissues may be improved. In the art, the immunogenicity of the collagen is mainly caused by the telopeptides. Treating the collagen with telopeptide hydrolases (such as pepsin, fig protease) can cut and remove the telopeptide to obtain the collagen without the telopeptide. However, immunochemical reactions of collagen implants are often caused by presence of cellular residues, protein impurity residues, fat residues, endotoxins, residues of extraction reagents, or chemical residues from cross-linking treatments. Collagens retained with telopeptides have not shown particular immune responses in clinical applications. In addition, the C-terminal telopeptide and the N-terminal telopeptide have important roles in cross-linking and fiber formation, and removal of the telopeptides often results in disordered arrangement of collagen molecules and loss of reconstructed collagen fiber patterns. In summary, the telopeptide region is biologically important for collagen materials.

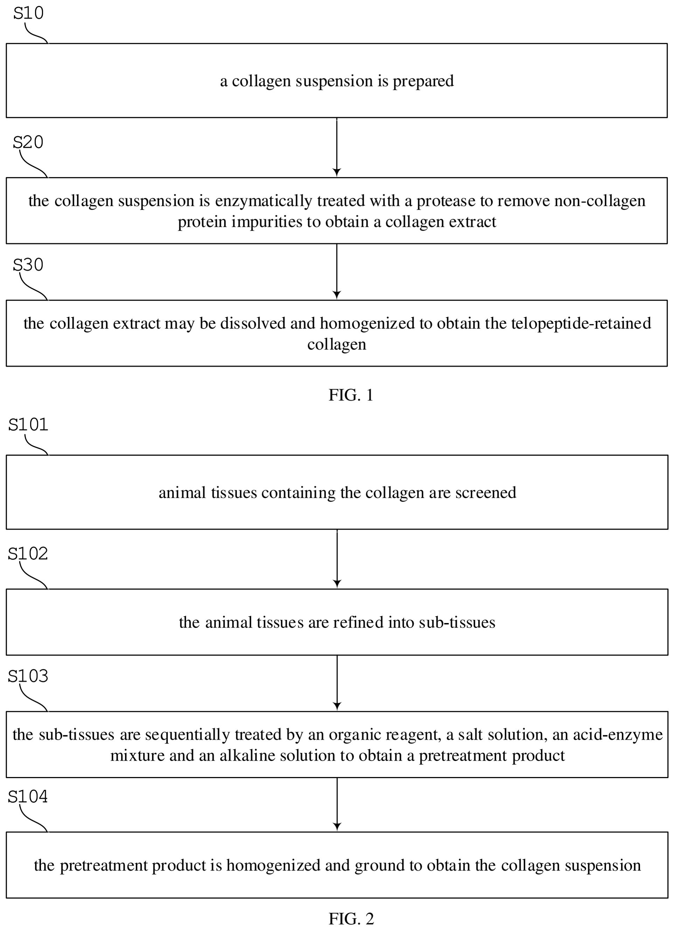

Therefore, the present disclosure provides a method for preparing the telopeptide-retained collagen. As shown in FIG. 1, the method for preparing the telopeptide-retained collagen provided by embodiments of the present disclosure may include following blocks.

In a block S10, a collagen suspension is prepared.

The collagen suspension may be prepared from an animal tissue containing collagen. In the collagen suspension, the collagen is in a form of particles, and the N-terminal peptide and the C-terminal peptide are retained.

Of course, in a case of having a finished collagen suspension, the block of preparing the collagen suspension in the present block may mean that the collagen suspension is obtained to be used at a later stage.

In a block S20, the collagen suspension is enzymatically treated with a protease to remove non-collagen protein impurities to obtain a collagen extract.

The protease has a higher enzymatic efficiency for the non-collagen protein impurities than for telopeptides of the collagen. The protease having the above properties may be combined with the non-collagen protein impurities more easily, and may have relatively fewer binding sites with the telopeptides. The protease has a relatively higher enzymatic activity for the non-collagen protein impurities and a relatively weaker enzymatic activity for the telopeptides. Therefore, while enzymatically degrading the non-collagen protein impurities, degradation of the telopeptides can be reduced, such that removal of the non-collagen protein impurities while retaining the telopeptides at the same time may be achieved.

In a block S30, the collagen extract may be dissolved and homogenized to obtain the telopeptide-retained collagen.

The collagen extract is dissolved to obtain a soluble telopeptide-retained collagen. Further, the soluble telopeptide-retained collagen is homogenized, such that the obtained telopeptide-retained collagen may further be dissolved, and impurities such as protein impurities in a product obtained from the block S20 may further be removed, and the telopeptide-retained collagen at higher purity may be obtained.

In the present disclosure, the protease is used to enzymatically hydrolyze the collagen suspension, the protease has the higher enzymatic efficiency for the non-collagen protein than that for the telopeptides of the collagen. In this way, degradation of the telopeptides can be reduced while the non-collagen protein impurities are degraded, such that while the non-collagen protein impurities and other impurities are removed, the telopeptides are retained. The prepared telopeptide-retained collagen has an intact triple helix structure, fewer non-collagen protein impurities, and a high telopeptide retention rate of more than 80%. Therefore, the method can be widely applied. The method for preparing the telopeptide-retained collagen of the present disclosure has simple and easy operations, the enzymatic reaction can be controlled, sources of the protease can be obtained, any equipment for performing conventional reaction conditions can be used. By performing the method of the present application, the telopeptide-retained collagen may be prepared in an industrialization scale, having significant economic values.

As shown in FIG. 2, in some embodiments, the block S10 of preparing the collagen suspension may include following blocks.

In a block S101, animal tissues containing the collagen are screened.

The animal tissues mainly refer to dermal tissues or Achilles tendon tissues that are preliminarily processed to contain only the collagen and have non-collagen protein impurities visible to naked eyes, such as epidermis, fat, fascia, and so on, removed. Animal sources include pigs, cows, sheep, horses, deer, and so on, an age thereof is generally less than 24 months, preferably 12 months-18 months.

The tissues may be inspected in accordance with requirements of the standard "YY/T 0771.2-2020 Animal-sourced medical devices, Part 2: Control of sources, collection and disposal". After confirmation of eligibility, fresh animal tissues are immersed in a disinfectant solution to be disinfected and cleaned. The disinfected and cleaned tissues are placed at -10°C to -40°C to be frozen and preserved, and frozen tissues are obtained.

The disinfectant solution includes peroxyacetic acid, sodium hypochlorite and polyhexamethylene bisguanidine, dodecyldimethylbenzylammonium chloride, and so on. For disinfecting and cleaning, the tissues are generally soaked in the above disinfectant solution for 5 min -10min and are then cleaned by purified water for 1 to 2 times.

In a block S102, the animal tissues are refined into sub-tissues.

The sub-tissues may be in various shapes, such as, in a blocked shape, in a thin-film shape, in a spherical shape, in a columnar shape, and so on. A size of the sub-tissues is in a range of 20 mm² to 1000 mm², such as 50 mm², 200 mm² and 500 mm², and so on. In a subsequent block S104, the sub-tissues will be further pre-treated, involving immersing the sub-tissues in a solution of organic reagents, salts, acids, bases, and enzymes. Therefore, the size of the sub-tissues affects a surface area with which the solution is in contact. For a fixed size of the animal tissues, as the size of the sub-tissues increases, a specific surface area decreases accordingly, and the surface area of which the above solutions contacts the sub-tissues decreases, a reaction rate is lower, and collagen compositions inside the sub-tissues may not be destroyed easily. Conversely, as the size of the sub-tissues decreases, the specific surface area increases accordingly, and the surface area of which the above solutions contacts the sub-tissues increases, the reaction rate is higher, and collagen compositions inside the sub-tissues may be destroyed more easily. Therefore, the size of the sub-tissues is limited to the above range, enabling the reaction rate of the sub-tissues in the subsequent solution to be controlled within a certain range, and the collagen compositions inside the sub-tissues may be protected.

Methods for refining the tissues include physical methods, such as crushing, pulverizing, churning, and cutting. For example, a bovine Achilles tendon tissue may be cut, by a cutting machine, into slices each having a thickness of 2 mm to 10 mm. A bovine dermal tissue is crushed into granules by a pulverizer, and the granules are screened by a 5 mm screen mesh. Alternatively, a porcine dermal tissue is churned to form 2 mm-10 mm tissue pieces using a tissue churning machine.

In the block S103, the sub-tissues are sequentially treated by an organic reagent, a salt solution, an acid-enzyme mixture and an alkaline solution to obtain a pretreatment product.

Sequential treatments remove impurities, such as protein impurities, fats, polysaccharides, microorganisms, endotoxins and viruses, from the sub-tissues.

In a block S104, the pretreatment product is homogenized and ground to obtain the collagen suspension.

The pretreatment product (i.e., the pretreated sub-tissues) obtained after the block S103 of cleaning and removing the impurities is loose and soft, and can be easily broken and ground. The above pretreatment product can be transferred to a closed circulating homogenizing and grinding apparatus, and the alkali solution can be added for homogenizing and grinding, so as to obtain the collagen suspension. A particle size of the suspension obtained after homogenization and grinding can be further reduced, allowing impurities, such as protein impurities to be fully exposed, and the amount of telopeptides of the collagen that is exposed may be appropriate. In this way, the enzymatic efficiency of the non-collagen protein impurities in an enzyme hydrolysis process subsequently may be improved, facilitating retention of the telopeptides of the collagen. Furthermore, centrifugal filtration, water-washing to remove protein impurities, and treatments of enzyme reagents after the enzymatic hydrolysis may also be facilitated, such that efficiencies of these operations may be increased.

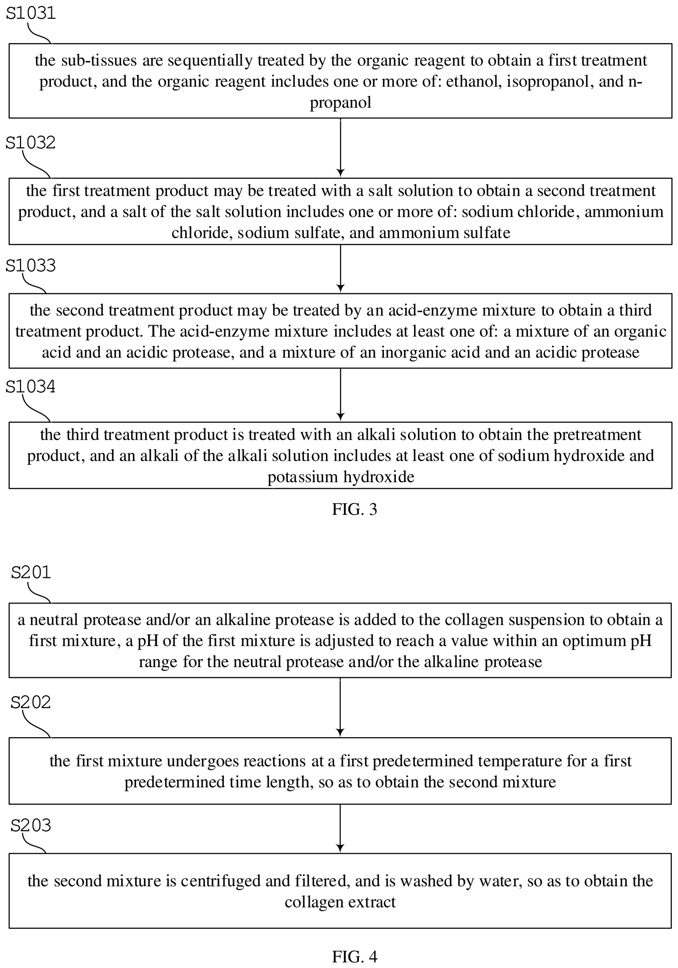

As shown in FIG. 3, in some embodiments, the block S103 where the sub-tissues are sequentially treated by the organic reagent, the salt solution, the acid-enzyme mixture and the alkaline solution to obtain a pretreatment product, may include following blocks.

In a block S1031, the sub-tissues are sequentially treated by the organic reagent to obtain a first treatment product, and the organic reagent includes one or more of: ethanol, isopropanol, and n-propanol.

The ethanol is both hydrophilic and hydrophobic, isopropanol is highly hydrophobic, and the n-propanol is highly hydrophilic. The organic reagent is one or more of: the ethanol, the isopropanol and the n-propanol. In this way, some inorganic salts, organic acids, fats, protein impurities and other impurities may be removed from the sub-tissues. The first treatment product in the present block, i.e., the sub-tissue treated with the organic reagent, retains an initial shape of the sub-tissue, such as in the blocked shape, in the thin-film shape, in the spherical shape, in the columnar shape, and the like.

In some embodiments, during the treatment with the organic reagent in the block S1031, a volume concentration of the organic reagent is, 60%-90% by volume, and a ratio of a weight of the sub-tissue to a weight of the organic reagent is 1:3-1:10; a temperature for the treatment is 25° C-30° C; a stirring speed during the treatment is 20rpm -60 rpm, and the treatment time is performed for 2h-3h.

The organic reagent may be an alcoholic reagent, which is one or more of the ethanol, the isopropanol and the n-propanol, and the volume concentration of the organic reagent may be, for example, 60%, 75%, 83%, 90% and the like. Specifically, the ethanol at the volume concentration of 75%, the n-propanol at the volume concentration of 90%, the isopropanol at the volume concentration of 60%; or a mixture of the ethanol at the volume concentration of 65%, the isopropanol at the volume concentration of 10%, and the n-propanol at the volume concentration of 25% may be used.

The ratio of the weight of the sub-tissue to the weight of the organic reagent is 1:3, 1:5, 1:7, 1:9, 1:10, and so on. The temperature for the treatment may be 25°C, 27°C, 29°C, 30°C, and so on. The stirring speed for the treatment may be 20 rpm, 40 rpm, 45 rpm, 60 rpm, and so on. A time length of the treatment may be 2h, 2.5h, 3h, and so on. The collagen may be denaturized when the concentration of the organic reagent being excessively high, a dosage thereof being excessively large, the temperature for the treatment being excessively high, and the stirring speed for the treatment being excessively high; and conversely, a decontamination effect may not be ideal. Therefore, the above parameters may be controlled within the above ranges, a better decontamination effect may be achieved.

In a block S1032, the first treatment product may be treated with a salt solution to obtain a second treatment product, and a salt of the salt solution includes one or more of: sodium chloride, ammonium chloride, sodium sulfate, and ammonium sulfate.

A sodium chloride solution, an ammonium chloride solution, a sodium sulfate solution and an ammonium sulfate solution may be used as the salt solution to remove some protein impurities, nucleic acids and the like from the sub-tissue. The second treatment product in the present block, i.e., the sub-tissue treated by the salt solution, retains the initial shape of the sub-tissue, such as being in the blocked shape, in the thin-film shape, in the spherical shape, in the columnar shape, and the like.

In some embodiments, during the treatment with the salt solution in the block S 1032, the salt solution may be a sodium chloride solution with a mass fraction of 5%-10%; or an ammonium chloride solution with a mass fraction of 5%-10%; or an ammonium sulfate solution with a mass fraction of 8%-12%; or a sodium sulfate solution with a mass fraction of 10%-14%. A ratio of the weight of the sub-tissue to a weight of the salt solution is 1:2-1:5. A temperature for the treatment is 20°C - 30°C. A stirring speed for the treatment is 10rpm -30 rpm. A time length of for the treatment is 4h-8 h. For example, the mass fraction of the sodium chloride solution may be 5%, 8%, 8.5%, 10%, and so on. The mass fraction of the ammonium chloride solution may be 5%, 7%, 8.5%, 10%, and so on. The mass fraction of the ammonium sulfate solution may be 5%, 6.5%, 9.5%, 10%, and so on. The mass fraction of the sodium sulfate solution may be 5%, 6.5%, 9%, 10% and so on. In the present block, the salt solution may be used to remove impurities such as protein impurities, nucleic acids, and so on. An effect of removing impurities may not be ideal when the concentration of the salt solution is excessively high or excessively low. Therefore, salt solutions within the above concentration ranges may be applied, so as to achieve a better impurity removal effect.

In a block S1033, the second treatment product may be treated by an acid-enzyme mixture to obtain a third treatment product. The acid-enzyme mixture includes at least one of: a mixture of an organic acid and an acidic protease, and a mixture of an inorganic acid and an acidic protease.

The third treatment product in the present block, i.e., the sub-tissue treated by the acid-enzyme mixture, retains the initial shape of the sub-tissue, such as being in the blocked shape, in the thin-film shape, in the spherical shape, in the columnar shape, and the like. For example, the second treatment product described in the above may continue being retained in a container, the acid-enzyme mixture may be added to soak the second treatment product, and the second treatment product soaked by the acid-enzyme mixture may be left standing. Subsequently, the soaked second treatment product may be washed with water to remove small debris, a pH of a washing solution may be adjusted to 6.0-7.0. The washed second treatment product may be filtered and collected to obtain the third treatment product. Acid-soluble proteins may be removed by the mixture of the organic acid and the acidic protease or the mixture of the inorganic acid and the acidic protease, and the second treatment product may further be swollen and loosened, and may be swollen by 3 to 5 times. A small amount of tissue debris may be generated during expansion and may be removed by washing with water.

To be noted that the acid solution may adjust the pH of a reaction system, such that the enzyme may be soluble in a relatively weak acidic environment and only maintains a certain activity, however, the adjusted pH of the reaction system may not be an optimal pH for the acidic proteases (e.g., pepsin) to perform activities. In the present block, although a small amount of telopeptides may be inevitably hydrolyzed, under limited reaction conditions, the enzyme is not intended to remove the telopeptides but rather to enable the tissue to be swollen and loosened, such that surface impurities may be removed. The tissue may be swollen for 3 to 5 times, accompanied by a small amount of tissue debris being generated, and the tissue debris may be removed by washing with water.

In some embodiments, during the treatment with the acid-enzyme mixture in the block S1033, a ratio of the weight of the second treatment product to a weight of the acid-enzyme mixture is 1:5-1:10, such as 1:5, 1:6, 1:8, 1:10. An acid of the acid-enzyme mixture may be acetic acid, citric acid, or hydrochloric acid, and an enzyme of the acid-enzyme mixture may be pepsin. The amount of the enzyme may be 1/5000-1/1000 of the weight of the second treatment product. A temperature of the treatment is 15° C -25° C, such as 15° C, 17° C, 21°C, 25°C, and so on. A time length of the treatment is 4h-8h, such as 4h, 6h, 8h, and so on. In the present block, the pH of the reaction environment in which the acidic protease is located is 5.0-7.0. That is, the acidic protease is in a weak acidic environment having limited activities and having limited hydrolytic activities for the telopeptides in the collagen of the second treatment product (i.e., the sub-tissue treated with the salt solution). In this way, most of the telopeptides are retained. Similarly, when the concentration of the acid solution is excessively high, structures of the telopeptides of the collagen may be destroyed; and when the concentration of the acid solution is excessively low, the impurity removal effect may not be ideal. When the concentration of the protease solution is excessively high, a highly effective cutting effect may be applied to the telopeptides; and the concentration of the protease solution is excessively low, the impurity removal effect may not be ideal. Therefore, the above parameters are controlled in the above ranges, the telopeptides may be retained, and the tissue may be swollen and loosened, and the surface impurities may be removed.

In some embodiments, during the treatment with the acid-enzyme mixture in the block S1033, the inorganic acid includes hydrochloric acid, and the organic acid includes at least one of acetic acid and citric acid; and the acidic protease includes pepsin and the like. The hydrochloric acid, the acetic acid, or a mixture of the hydrochloric acid and the acetic acid may be used. The acid solution may adjust the pH of the reaction system, such that the enzyme is in the weak acidic environment and an activity thereof is inhibited.

In some embodiments, during the treatment with the acid-enzyme mixture in the block S1033, the ratio of the weight of the enzyme solution to the weight of the second treatment product is 1:5000-1:1000. For example, the ratio of the weight of the enzyme solution to the weight of the second treatment product may be 1:5000, 1:4000, 1:3500, 1:3000, 1:2500, 1:2000, 1:1500, 1. 1000, and so on. As described in the above, the excessively high concentration of the enzyme solution may perform excessively strong cutting effect on the telopeptides, and the excessively low may cause the poor impurity removal effect. Therefore, the ratio of the weight of the enzyme solution to the weight of the second treatment product is controlled in the above range, such that the telopeptide is retained, and the impurities are removed at the same time.

In a block S1034, the third treatment product is treated with an alkali solution to obtain the pretreatment product, and an alkali of the alkali solution includes at least one of sodium hydroxide and potassium hydroxide.

Since acid may be left in the third treatment product from the block S1033, after adding the alkali solution in the present block, the third treatment product shrinks. However, as the alkali neutralizes the acid, the third treatment product may be swollen by 2 to 3 times, and tissue debris may be generated, which may be removed by washing with water. When the debris are removed by washing with water until a pH of the washing solution reaching 7.0-8.5, the third treatment product may be filtered and collected to obtain the pretreatment product.

In addition, in the present block, some alkali-soluble protein impurities may be removed.

In some embodiments, in a process of treating the third treatment product with the alkali solution in the block S1034, a mass fraction of the alkali solution is 0.1 M-0.5 M, a ratio of a weight of the third treatment product to a weight of the alkali solution is 1:5-1:8. A temperature for the treatment is 4° C -18° C. A time length of the treatment is 8 h -12 h. For example, the mass fraction of the alkali solution may be 0.1M, 0.3M, 0.4M, 0.5 M, and so on. The ratio of the weight of the third treatment product to the weight of the alkali solution may be 1:5, 1:65, 1:8, and so on. The temperature for the treatment is 4°C, 8°C, 15°C, 18°C, and so on. The time length of the treatment is 8 h, 10 h, 12 h, and so on. When the concentration of the alkali solution is excessively high, the structure of the collagen may be destroyed. When the concentration of the alkali solution is excessively low, the impurity removal effect may not be ideal. When the ratio of the weight of the third treatment product to the weight of the alkali solution is excessively high, a content of the alkali solution may be small, the impurity removal effect may not be ideal. When the temperature for the treatment is excessively high, the structure of the collagen may be destroyed. When the time length of the treatment is excessively short, the impurity removal effect may not be ideal. Therefore, the above parameters are controlled in the above ranges, the structure of the collagen is protected, and the impurity removal effect may be ideal.

In some embodiments, the block S104 where the pretreatment product is homogenized and ground to obtain the collagen suspension includes following blocks.

In a block S1041, the pretreatment product is transferred to a homogenizing and grinding device, an alkaline solution is added, and the pretreatment product is homogenized and ground to have a particle size of 20 µm -400 µm, so as to obtain the collagen suspension.

By adding the alkaline solution, the tissue may be in a loosened state. By homogenizing and grinding the pretreatment product to have the particle size of 20 µm -400 µm, the particle size of the pretreatment product may be appropriate, such that the enzymatic efficiency of the non-collagen protein impurities in the subsequent enzymatic hydrolysis process may be improved, and an ideal retention rate of the telopeptides of the collagen may be obtained. In addition, centrifugation and filtration, washing to remove the protein impurities and enzyme reagents, and other operations after the enzymatic hydrolysis may all be facilitated, and the efficiency may be improved.

A pH of the added alkaline solution is 8.0-10.0, such as 8.0, 8.5, 9.5, 10.0, and so on.

A temperature of the process is 15° C -25° C. The particle size of the collagen in the collagen suspension is 20 µm -400 µm. The temperature of the process may be 15° C, 18° C, 22° C, 25° C, and so on. The particle size of the collagen in the collagen suspension may be 20 µm, 80 µm , 100 µm, 160 µm, 200 µm, 250 µm, 320 µm, 400 µm, and the like.

Since the acid-enzyme mixture is used for treatment in the previous block S103, the pH of the reaction system is acidic, the pH of the pretreatment product can be adjusted in the present block by using sodium hydroxide solution or potassium hydroxide solution or the like. In this way, the pH of the pretreatment product may be between 8.0 and 10.0, and therefore, some alkali-soluble protein impurities can be removed. When the temperature of the process is excessively high, the collagen may be denaturized; and when the temperature of the process is excessively low, the impurity removal effect may not be ideal. When the particle size is excessively large, a specific surface area is excessively small, and the pretreatment product may not be in contact with other solutions properly in subsequent treatments. When the particle size is excessively small, the specific surface area is excessively large, the structure of the collagen may be destroyed easily. Therefore, the above parameters are controlled in the above ranges, the structure of the collagen may be protected, and the impurities may be removed effectively.

As shown in FIG. 4, in some embodiments, the block S20 where the collagen suspension is enzymatically treated with the protease to remove non-collagen protein impurities to obtain the collagen extract includes following blocks.

In a block S201, a neutral protease and/or an alkaline protease is added to the collagen suspension to obtain a first mixture, a pH of the first mixture is adjusted to reach a value within an optimum pH range for the neutral protease and/or the alkaline protease.

On the one hand, by adding the neutral protease and/or the alkaline protease to the collagen suspension and adjusting the pH of the first mixture to the optimum pH range for the neutral protease and/or alkaline protease, the pH of the first mixture is neutral or alkaline, swelling under acidic conditions may not occur, more telopeptide cleavage sites may not be exposed, a state of the collagen suspension and the molecular structure of the collagen may be stabilized, hydrolysis of the telopeptides may be reduced, and the telopeptides may be retained at the collagen. On the other hand, by using the neutral protease and/or the alkaline protease, the neutral protease and/or the alkaline protease may have different enzymatic efficiencies for different substrates. The neutral protease and/or the alkaline protease are more likely to combine with non-collagen protein impurities and have relatively fewer binding sites with the telopeptides. The neutral protease and/or the alkaline protease exhibit relatively high enzymatic activities for the non-collagen protein impurities and relatively weak activities for the telopeptides. Therefore, degradation of the telopeptides may be reduced while the non-collagen protein impurities are removed by effective enzymatic hydrolysis. In this way, the telopeptides are retained while the non-collagen protein impurities and other impurities are removed, and a high retention rate of telopeptides in the collagen may be obtained.

It is to be noted that the neutral protease does not mean a class of proteases having the optimal activities at the pH of 7.0, but refers to a protease which has good activities at neutral pH values, neutral-weakly acidic pH values or neutral-weakly alkaline pH values.

Specifically, a pH range for the neutral protease in the present embodiment to have optimal activities may be in a range of 6 to 8, such as 6.0, 6.5, 6.7, 7.1, 7.3, 7.6, or 8.0.

Specifically, a pH range for the alkaline protease in the present embodiment to have optimal activities may be in a range of 7-10.

The collagen suspension may be treated with the neutral protease alone, or the alkaline protease alone, or a mixture of both the neutral protease and the alkaline protease.

It can be seen that the pH range of the protease in the present embodiment to have optimal activities is weak acidic, neutral, or weak alkaline. In this case, the activity of the protease is moderate, destruction of the telopeptide may be reduced while the non-collagen protein impurities are effectively enzymatically hydrolyzed and removed. Moreover, the state of the collagen suspension and the molecular structure of the collagen can be stabilized when the reaction environment is within the above pH range. The collagen suspension may not be swollen due to acidic conditions and may not expose more telopeptide cleavage sites. In this way, the state of the collagen suspension and the molecular structure of the collagen can be stabilized, hydrolysis of the telopeptides may be reduced, and the telopeptides may be retained at the collagen.

In addition, the enzyme used in the present embodiment may be derived from microorganisms, such as bacteria, actinomycetes, and molds, and so on. The enzyme may be serine protein hydrolases of endopeptidases and may catalyze hydrolysis of amino acid amides and ester bonds of proteins. The enzyme used in the present embodiment may hydrolyze certain proteins into peptides or amino acids, essential catalytic active sites may be serine, histidine, aspartic acid, and alanine.

Specifically, the protease includes one or more of, for example, *Bacillus subtilis* protease, *Bacillus licheniformis* protease, *Aspergillus oryzae* protease, *Streptomyces griseus* protease, and *Fusarium* protease.

The amount of protease may be related to a weight of the animal tissue from which the collagen suspension is prepared. When at least one of *Bacillus licheniformis* alkaline protease or *Aspergillus oryzae* alkaline protease is used, a ratio of a weight of the protease to the weight of the animal tissue from which the collagen suspension is prepared is 1:500-1:50, such as 1:500, 1:450, 1:350, 1:300, 1:250, 1:200, 1:100, 1:50, 1:20, and so on. When at least one of *Bacillus subtilis* alkaline protease, *Streptomyces griseus* alkaline protease, or *Fusarium* alkaline protease is used, the ratio of the weight of the protease to the weight of the animal tissue from which the collagen suspension is prepared is 1:100-1:20, such as 1:100, 1:75, 1:50, 1:20, and so on.

In some embodiments, in the block S201, when the neutral protease and/or the alkaline protease is added to the collagen suspension, an enzyme stabilizer may further be added to the collagen suspension, so as to obtain the first mixture. The enzyme stabilizer may stabilize and protect the enzyme to enable the enzyme to perform enzymatic reactions stably.

Specifically, the enzyme stabilizer includes at least one of propylene glycol, polyethylene glycol, sucrose, and mannitol.

In order to protect the telopeptides, in some embodiments, before treating the collagen suspension with the protease, the method further includes following blocks.

In a block S200, a collagen terminal-group protector is added to the collagen.

The collagen terminal-group protector is added before the protease performs the enzymatic reaction, so as to protect the telopeptides from being hydrolyzed during the enzymatic reaction. In this way, the telopeptides of the collagen are further protected, and the retention rate of the telopeptides of the extracted collagen may be increased.

Specifically, the collagen terminal-group protector includes at least one of a highly unsaturated fatty acid and an amino acid. The highly unsaturated fatty acid includes at least one of arachidonic acid, eicosapentaenoic acid, eicosapentaenoic acid, docosapentaenoic acid, docosahexaenoic acid, linoleic acid, and linolenic acid. The highly unsaturated fatty acid produces a dilute aldehyde and some peroxides under oxidizing conditions. The peroxides may easily bind to a carboxyl group or an amino group of the telopeptide of the collagen fiber, enabling the telopeptide to resist against enzymatic hydrolysis, such that the telopeptide can be retained. The amino acid includes at least one of glycine/polyglycine, lysine, polylysine, glutamic acid and polyglutamic acid. The amino acid can bind to an exposed carboxyl group or amino group of the telopeptide to form an amide compound to protect the telopeptide from being enzymatically hydrolyzed.

In a block S202, the first mixture undergoes reactions at a first predetermined temperature for a first predetermined time length, so as to obtain the second mixture.

The first mixture undergoing reactions at the first predetermined temperature for the first predetermined time length of time allows the protease to comprehensively enzymatically hydrolyze the impurities such as the non-collagen protein impurities
Specifically, the first predetermined temperature is 4° C to 20° C, such as 4° C, 7° C, 10° C, 16° C, or 20° C, and the like.

By controlling the temperature for the protease to perform enzymatic hydrolysis at the first predetermined temperature, the state of the collagen suspension and the molecular structure of the collagen can be stabilized, the triple helix structure of the collagen can be protected, and activities of the protease can be better moderately controlled to reduce activities of the protease in cleaving the telopeptide.

Specifically, the first predetermined time length is 4h-8h, such as 4h, 5.5h, 7.2h or 8h, and the like. During the first mixture undergoing reactions, the first mixture may be stirred. The stirring may allow the protease to be in fully contact and to react with the collagen in the collagen suspension, such that the impurities such as the non-collagen protein impurities can be fully removed.

The stirring speed is 10 rpm -20 rpm, such as 10 rpm, 15 rpm or 20 rpm. Specifically, in the block S201, under a clean environment, the collagen suspension may be transferred to a stir-reaction container, and the protease is added. Therefore, the first mixture may be stirred by the stir-reaction container.

In a block S203, the second mixture is centrifuged and filtered, and is washed by water, so as to obtain the collagen extract.

The impurities, formed after the enzymatic hydrolysis, and the used protease in the block S202 need to be separated from the collagen.

Centrifugation and filtration and water washing may refer to continuously applying water to achieve washing and centrifugation being performed simultaneously. In this way, due to the water-soluble property of the protease and the water-soluble property of small molecule impurities, such as protein impurities obtained from enzymatic hydrolysis, and due to a centrifugal force generated during the centrifugation, the water-soluble impurities may be removed efficiently, rapidly, and thoroughly, and large-sized particles of the collagen suspension are retained.

Specifically, the separation may be performed by repetitive water-washing and filtration and repetitive centrifugation filtration. In some embodiments, in the block S203, the second mixture is centrifuged and filtered and washed by water, so as to obtain the collagen extract. A rotation speed of the centrifugation is 2000 rpm to 4000 rpm. A volume of the water for washing is 3 to 5 times of a volume of the second mixture. For example, the rotation speed of the centrifugation may be 2000 rpm, 2500 rpm, 2800 rpm, 3500 rpm, 3800 rpm, 4000 rpm, and the like. The volume of the water for washing may be 3 times, 3.5 times, 4 times, 4.5 times, 5 times, and the like of the volume of the second mixture. By controlling the rotation speed of the centrifugation and the volume of the water for washing within the above ranges, the impurities may be fully precipitated, some salts, organic reagents and other impurities may be sufficiently removed, so as to obtain the collagen with higher purity.

In some embodiments, the block S203 where the second mixture is centrifuged and filtered and is washed by water so as to obtain the collagen extract, includes following blocks.

In a block S2031, the second mixture is placed in a device in which centrifugation, filtration, and water washing may be performed at the same time, and the device is arranged with a filter bag, and a hole size of the filter bag is 800 mesh-1000 mesh.

In the block S202, the first mixture is stirred, and the obtained second mixture contains the collagen, weak alkaline proteases and/or protein impurities obtained by being hydrolyzed by the weak alkaline proteases, and other impurities. By performing centrifugation and precipitation, target collagen is obtained, a supernatant containing impurities is removed. By performing the filtration, other impurities having molecular weights different from that of the collagen may be removed. The hole size of the filter bag may be 800 mesh, 850 mesh, 900 mesh, 950 mesh, 1,000 mesh and the like. Water-soluble proteins, organic reagents and other impurities may be removed by water washing.

The blocks S201 to S204 of the present disclosure are significantly critical for retaining the telopeptides. Only under specific conditions as limited in the present disclosure, such as specific temperatures for the enzymatic hydrolysis, the specific time length of the enzymatic hydrolysis, and the specific dosages of the proteases, the telopeptides of the collagen can be retained while the non-collagen protein impurities and other impurities are removed, and a high rate of retention of the telopeptides can be achieved. The collagen extract obtained after being treated by the proteases is a delicate, pliable and smooth white solid product.

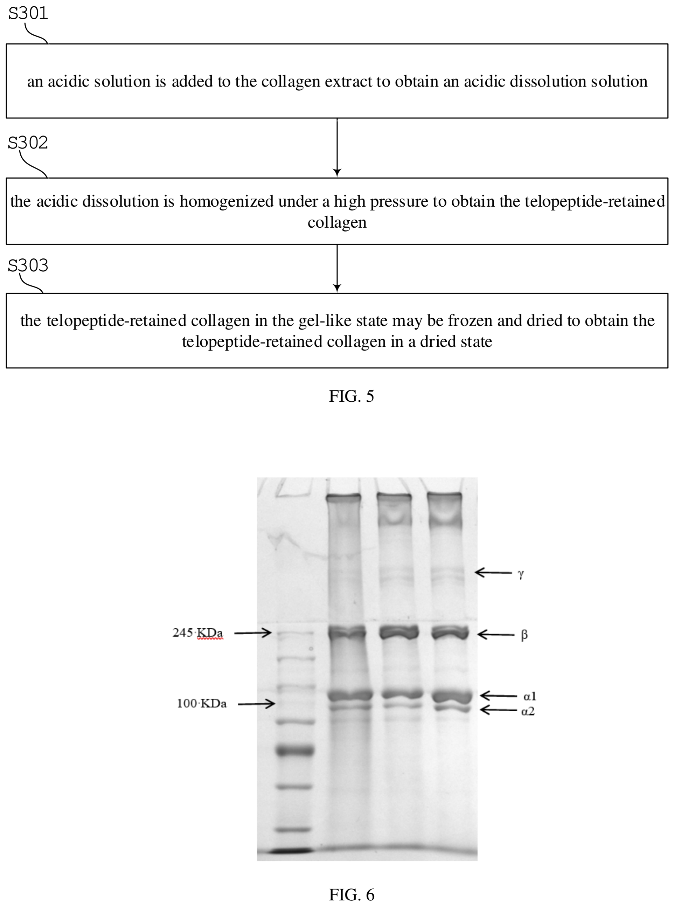

As shown in FIG. 5, in some embodiments, the block S30 where the collagen extract is dissolved and homogenized to obtain the telopeptide-retained collagen, includes following blocks.

In a block S301, an acidic solution is added to the collagen extract to obtain an acidic dissolution solution.

Adding the acidic solution to the collagen extract improves a dissolution efficiency of the collagen, such that the collagen suspension after being enzymatically hydrolyzed can be further dissolved and solubilized, and solubilized telopeptide-retained collagen can be obtained.

The acidic solution includes at least one of acetic acid, hydrochloric acid, sulfuric acid or citric acid.

Specifically, the acidic solution may be acetic acid, which has a good dissolution solubilization effect. The collagen extract may be placed in an airtight container. A mass ratio of the collagen extract to the acidic solution may be 1:5-1:10. The acidic solution may be a 5 mM-10 mM acetic acid solution. A stirring temperature may be 20°C-25°C. A stirring rotational speed may be 10 rpm -15 rpm. After stirring, most of the collagen extract in a solid state may be dispersed and dissolved into a gel-like state under the acid solution system.

Of course, in other embodiments, instead of adding the acidic solution, purified aqueous solution or PBS buffer solution (pH 6.0-7.5) may be used for extraction. In the present embodiment, by using the acidic solution, an efficiency of dissolution to extract the collagen may be high.

In a block S302, the acidic dissolution is homogenized under a high pressure to obtain the telopeptide-retained collagen.

The high pressure for homogenization may further facilitate dissolution and extraction of the telopeptide-retained collagen. Specifically, a high-pressure fluid homogenizer may be used. Homogenization conditions may be: one homogenization at a pressure of 200 bar-400 bar, followed by another homogenization at a pressure of 400 bar-600 bar, and a homogenization temperature of 10°C-18°C, such that the telopeptide-retained collagen in the gel-like state may be obtained.

In some embodiments, other forms of the telopeptide-retained collagen may alternatively be obtained. The block S30 where the collagen extract is dissolved and homogenized to obtain the telopeptide-retained collagen, further includes following blocks.

In a block S303, the telopeptide-retained collagen in the gel-like state may be frozen and dried to obtain the telopeptide-retained collagen in a dried state.

Freezing and drying may be performed by a low-temperature drying method known in the art. A purpose of the freezing and drying is to remove organic acids and water from the collagen in the gel-like state, so as to obtain the collagen in the dried state, which may be preserved easily.

Embodiments of the present disclosure further provide the telopeptide-retained collagen prepared based on the above methods. The telopeptide-retained collagen provided by the embodiments of the present disclosure is prepared by combination of mild physical, chemical and enzymatic methods. The non-collagen impurities, microorganisms, endotoxins, potential viruses, protein impurities, inorganic salts, organic solvents, and other impurities are removed, and at the same time, the telopeptide-retained collagen for medical applications is obtained. Being different from the art, the telopeptide-retained collagen provided by the embodiments of the present disclosure is different from the telopeptide-removed collagen in the art, materialogical defects of the telopeptide-removed collagen, including fewer cross-linking sites, reduced properties for forming fibers, poor thermal stability, poor mechanical properties, and poor in-vitro selfassembling/gel properties, may be overcome. The telopeptide-retained collagen of the present application provides more sites for intermolecular cross-linking, and can be more widely applied for tissue engineering products which may have requirements for having higher structural integrity, strength, and stiffness. In addition, the telopeptide-retained collagen provided by the embodiments of the present disclosure may have an intact triple helix structure, impurities may be more thoroughly removed, and the telopeptide-retained collagen may be in a high purity. The preparation method is simple, and in particular, for purification, tedious and complicated steps, such as saline dialysis, dialysis, ultrafiltration, ion exchange chromatography, and so on, are not performed. Therefore, an extraction period is shorter, costs are lower, and industrial-scale production can be achieved.

Technical effects of the present disclosure will be further described below by referring to embodiments.

In order to make the technical problems, technical solutions and technical effects of the embodiments of the present disclosure to be clearer, the present disclosure will be further described in detail by referring to embodiments and the accompanying drawings. Apparently, the described embodiments are only a part of, not all of, the embodiments of the present disclosure. Following description of at least one exemplary embodiment is merely illustrative and does not form any limitation of the present disclosure and applications thereof. All other embodiments, which are obtained by any ordinary skilled person in the art based on the embodiments in the present disclosure without creative work, shall fall within the scope of the present disclosure.

### 1. Embodiments

### Embodiment 1

1. Animal tissue selection: a fresh 18-month-old bovine dermal tissue is examined, and an appearance, a color and an odor thereof are confirmed. A qualified bovine dermal tissue is immersed into 0.15% peroxyacetic acid solution for 8 min, then washed by purified water for 2 times, and then placed in a -18° C freezer to be used later.
2. Tissue refinement: the frozen dermal tissue is taken out. The dermal tissue in the frozen state is crushed by a universal crusher. The crushed dermal tissue is filtered through a 3cm stainless steel filter mesh. Tissue fragments having a particle size of 3cm or less are obtained.
3. Cleaning and impurity removal:
   3.1 Treatment with an organic reagent: in a clean workshop, the tissue fragments are placed into the stir-reaction container, 75% ethanol solution in a weight of 3 times of a weight of the tissue fragments is added to the container. A temperature of the container is set at 28°C, a stirring speed is set at 40rpm. After 3h treatment, the solution is discarded, the tissue fragments are collected by the filter mesh in the stir-reaction container, and an equal amount of purified water is added to the container for cleaning, and the cleaning is performed for 2 times.
   3.2 Salt solution treatment: in a clean workshop, a 7.5% sodium chloride solution in a weight of 5 times of the weight of the tissue fragments is added into the stir-reaction container. A temperature of the container is set at 25°C, a stirring speed is set at 30rpm. After 8h treatment, the solution is discarded, the tissue fragments are collected by the filter mesh in the stir-reaction container, and an equal amount of purified water is added to the container for cleaning, and the cleaning is performed for 2 times.
   3.3 Acid-enzyme mixture treatment: in a clean workshop, a 0.5M acetic acid solution in a weight of 5 times of the weight of the tissue fragments and pepsin in a weight of 1/1000 of the weight of the tissue fragments are added into the stir-reaction container. A temperature of the container is set at 20°C. The container is left undisturbed. After 8h treatment, the solution is discarded, the tissue fragments are collected by the filter mesh in the stir-reaction container, and purified water is added to the container for cleaning until a pH of a cleaning solution reaches 6.0-7.0.
   3.4 Alkaline solution treatment: in a clean workshop, a 0.5M sodium hydroxide solution in a weight of 8 times of the weight of the tissue fragments is added into the stir-reaction container. A temperature of the container is set at 4°C. The container is left undisturbed. After 12h treatment, the solution is discarded, the tissue fragments are collected by the filter mesh in the stir-reaction container, and purified water is added to the container for cleaning until a pH of a cleaning solution reaches 7.0-8.5.
4. Homogenization and grinding: in a clean workshop, the tissue fragments, after being cleaned and having the impurities removed, are taken out and transferred to a circulating homogenization and grinding device. A sodium hydroxide solution having a pH value of 9.0 and in a weight of 10 times of the weight of the tissue fragments is added. A temperature of the container is set at 15°C. The tissue fragments are ground into the collagen suspension.
5. Extraction and purification: in a clean workshop, the above collagen suspension is transferred to the stir-reaction container, an equal volume of purified water is added to the stir-reaction container, and then a *Bacillus subtilis* alkaline protease in a weight of 1/100 of the weight of the tissue fragments is added to the stir-reaction container. A temperature of the container is set at 15°C. A stirring speed is set to 20 rpm, stirring for 6h. Subsequently, the extract is water washed and filtered at the same time through a centrifuge. A hole size of the filter bag is 1000 mesh, and a centrifugation speed is 4000 rpm.
6. Dissolution and homogenization: in a clean workshop, the collagen extract obtained after centrifugation is placed in the stir-reaction container. A 10mM acetic acid solution having a weight of 10 times of a weight of the collagen extract is added to the stir-reaction container. A temperature of the container is set at 25°C. A stirring speed is set to 15 rpm, stirring dissolution for 12h. The acid dissolution is processed by a high-pressure fluid homogenizer for once in a pressure of 200 bar and for once in a pressure of 400bar. A homogenization temperature is 18°C. The collagen in the gel-like state is collected and obtained.
7. Freezing and drying: the collagen in the gel-like state is frozen and dried to obtain the collagen in the solid state.

### Embodiment 2

1. Animal tissue selection: a fresh 12-month-old porcine dermal tissue is examined, and an appearance, a color and an odor thereof are confirmed. A qualified porcine dermal tissue is immersed into 0.08% sodium hypochlorite acid solution for 5 min, then washed by purified water for 2 times, and then placed in a -40° C freezer to be used later.
2. Tissue refinement: the frozen dermal tissue is taken out. The dermal tissue in the frozen state is crushed by a universal crusher, so as to obtain tissue fragments having a particle size of 3cm to 4cm.
3. Cleaning and impurity removal:
   3.1 Treatment with an organic reagent: in a clean workshop, the tissue fragments are placed into the stir-reaction container, 60% isopropyl alcohol solution in a weight of 10 times of a weight of the tissue fragments is added to the container. A temperature of the container is set at 30°C, a stirring speed is set at 60rpm. After 2h treatment, the solution is discarded, the tissue fragments are collected by the filter mesh in the stir-reaction container, and an equal amount of purified water is added to the container for cleaning for once.
   3.2 Salt solution treatment: in a clean workshop, a 10% sodium sulfate solution in a weight of 4 times of the weight of the tissue fragments is added into the stir-reaction container. A temperature of the container is set at 30°C, a stirring speed is set at 30rpm. After 4h treatment, the solution is discarded, the tissue fragments are collected by the filter mesh in the stir-reaction container, and an equal amount of purified water is added to the container for cleaning, and the cleaning is performed for 3 times.
   3.3 Acid-enzyme mixture treatment: in a clean workshop, a 0.01M hydrochloric acid solution in a weight of 10 times of the weight of the tissue fragments and pepsin in a weight of 1/5000 of the weight of the tissue fragments are added into the stir-reaction container. A temperature of the container is set at 15°C. The container is left undisturbed. After 4h treatment, the solution is discarded, the tissue fragments are collected by the filter mesh in the stir-reaction container, and purified water is added to the container for cleaning until a pH of a cleaning solution reaches 6.0-7.0.
   3.4 Alkaline solution treatment: in a clean workshop, a 0.5M sodium hydroxide solution in a weight of 8 times of the weight of the tissue fragments is added into the stir-reaction container. A temperature of the container is set at 4°C. The container is left undisturbed. After 8h treatment, the solution is discarded, the tissue fragments are collected by the filter mesh in the stir-reaction container, and purified water is added to the container for cleaning until a pH of a cleaning solution reaches 7.0-8.5.
4. Homogenization and grinding: in a clean workshop, the tissue fragments, after being cleaned and having the impurities removed, are taken out and transferred to a circulating homogenization and grinding device. A sodium hydroxide solution having a pH value of 10.0 and in a weight of 10 times of the weight of the tissue fragments is added. A temperature of the container is set at 25°C. The tissue fragments are ground into the collagen suspension.
5. Extraction and purification: in a clean workshop, the above collagen suspension is transferred to the stir-reaction container, purified water having a volume of 1.5 times of the collagen suspension is added to the stir-reaction container, and then a *Bacillus licheniformis* alkaline protease in a weight of 1/500 of the weight of the sub-tissue is added to the stir-reaction container. A temperature of the container is set at 18°C. A stirring speed is set to 20 rpm, stirring for 8h. Subsequently, the extract is water washed and filtered at the same time through a centrifuge. A hole size of the filter bag is 800 mesh, and a centrifugation speed is 3000 rpm.
6. Dissolution and homogenization: in a clean workshop, the collagen extract obtained after centrifugation is placed in the stir-reaction container. A 5 mM acetic acid solution having a weight of 5 times of a weight of the collagen extract is added to the stir-reaction container. A temperature of the container is set at 20°C. A stirring speed is set to 10 rpm, stirring dissolution for 24h. The acid dissolution is processed by a high-pressure fluid homogenizer for once in a pressure of 400 bar and for once in a pressure of 600bar. A homogenization temperature is 10°C. The collagen in the gel-like state is collected and obtained.
7. Freezing and drying: the collagen in the gel-like state is frozen and dried to obtain the collagen in the solid state.

### Embodiment 3

1. Animal tissue selection: a fresh 18-month-old bovine Achilles tendon tissue is examined, and an appearance, a color and an odor thereof are confirmed. A qualified porcine dermal tissue is immersed into 0.16% polyhexamethylene biguanide (PHMB) for 5 min, then washed by purified water for 2 times, and then placed in a -40° C freezer to be used later.
2. Tissue refinement: the frozen bovine Achilles tendon tissue is taken out and is cut by a cutter into thin tissue slices, each having a thickness of 2cm to 4cm. The thin tissue slices are collected to be used later.
3. Cleaning and impurity removal:
   3.1 Treatment with an organic reagent: in a clean workshop, the thin tissue slices are placed into the stir-reaction container, a mixed alcohol solution (65% ethanol: 10% isopropanol : 15% n-propanol mixture : 10% purified water) in a weight of 3 times of a weight of the thin tissue slices is added to the container. A temperature of the container is set at 25°C, a stirring speed is set at 20rpm. After 3h treatment, the solution is discarded, the thin tissue slices are collected by the filter mesh in the stir-reaction container, and an equal amount of purified water is added to the container for cleaning for 2 times.
   3.2 Salt solution treatment: in a clean workshop, a 12% sodium sulfate solution in a weight of 5 times of the weight of the thin tissue slices is added into the stir-reaction container. A temperature of the container is set at 20°C, a stirring speed is set at 30rpm. After 6h treatment, the solution is discarded, the thin tissue slices are collected by the filter mesh in the stir-reaction container, and an equal amount of purified water is added to the container for cleaning, and the cleaning is performed for 2 times.
   3.3 Acid-enzyme mixture treatment: in a clean workshop, a 0.1M acetic acid solution in a weight of 5 times of the weight of the thin tissue slices and pepsin in a weight of 1/2000 of the weight of the thin tissue slices are added into the stir-reaction container. A temperature of the container is set at 20°C. The container is left undisturbed. After 4h treatment, the solution is discarded, the thin tissue slices are collected by the filter mesh in the stir-reaction container, and purified water is added to the container for cleaning until a pH of a cleaning solution reaches 6.0-7.0.
   3.4 Alkaline solution treatment: in a clean workshop, a 0.2M sodium hydroxide solution in a weight of 5 times of the weight of the thin tissue slices is added into the stir-reaction container. A temperature of the container is set at 18°C. The container is left undisturbed. After 12h treatment, the solution is discarded, the thin tissue slices are collected by the filter mesh in the stir-reaction container, and purified water is added to the container for cleaning until a pH of a cleaning solution reaches 7.0-8.5.
4. Homogenization and grinding: in a clean workshop, the thin tissue slices, after being cleaned and having the impurities removed, are taken out and transferred to a circulating homogenization and grinding device. A sodium hydroxide solution having a pH value of 8.0 and in a weight of 15 times of the weight of the sub-tissue is added. A temperature of the container is set at 15°C. The thin tissue slices are ground into the collagen suspension.
5. Extraction and purification: in a clean workshop, the above collagen suspension is transferred to the stir-reaction container, an equal amount of purified water is added to the stir-reaction container, and then a *Fusarium* alkaline protease in a weight of 1/60 of the weight of the thin tissue slices is added to the stir-reaction container. A temperature of the container is set at 10°C. A stirring speed is set to 15 rpm, stirring for 6h. Subsequently, the extract is water washed and filtered at the same time through a centrifuge. A hole size of the filter bag is 800 mesh, and a centrifugation speed is 4000 rpm.
6. Dissolution and homogenization: in a clean workshop, the collagen extract obtained after centrifugation is placed in the stir-reaction container. A 7.5 mM acetic acid solution having a weight of 7.5 times of a weight of the collagen extract is added to the stir-reaction container. A temperature of the container is set at 25°C. A stirring speed is set to 15 rpm, stirring dissolution for 18h. The acid dissolution is processed by a high-pressure fluid homogenizer for 2 times in a pressure of 400 bar. A homogenization temperature is 15°C. The collagen in the gel-like state is collected and obtained.
7. Freezing and drying: the collagen in the gel-like state is frozen and dried to obtain the collagen in the solid state.

### 2. Performance tests

According to the medical standard "YY/T 1453-2016 Tissue Engineering Medical Device Product Type I Collagen Characterization Methods", an amount of the protein impurities, an amount of the collagen, an amount of hydroxyproline, a total amount of polysaccharides, an amount of fat, microbial limits, an amount of endotoxin are tested. ELISA is performed to detect an amount of the N-terminal telopeptide and an amount of the C-terminal telopeptide.

**Table 1 Methods for testing performances**

| Tested items | Method for testing |
|---|---|
| Amount of proteins (%) | Spectrophotometry |
| Amount of protein impurities (%) | SDS-PAGE |
| amount of | UV Spectrophotometry |
| hydroxyproline (%) | |
| total amount of polysaccharides (%) | Colorimetric Analysis |
| amount of fat (%) | Acid Hydrolysis Distillation |
| microbial limits (CFU/g) | "Chinese Pharmacopoeia" 1105 Microbial Limit Inspection Method for Non-sterile Products |
| amount of endotoxin (EU/ ml) | Dynamic Chromogenic Method |
| retention rate of telopeptides (%) | ELISA |
| N-terminal telopeptide | |
| C-terminal telopeptide | |

As shown in Table 2, for comparison with embodiments of the present disclosure, the applicant purchased commercial bovine Achilles tendon type I collagen material, which is tested for corresponding items. The commercial bovine Achilles tendon type I collagen material is a telopeptide-removed collagen extracted by using combination of acid and protein hydrolases to remove the telopeptides.

**Table 2 Test results of the Embodiment 1, the Embodiment 2, the Embodiment 3 and the commercial collagen**

| Tested items | Embodiment **1** | Embodiment **2** | Embodiment **3** | commercial collagen |
|---|---|---|---|---|
| Amount of protein impurities (%) | 99.10% | 98.80% | 99.50% | 95.30% |
| Amount of protein impurities (%) | 0.25% | 0.50% | 0.25% | 0.75% |
| amount of hydroxyproline (%) | 12.20% | 12.40% | 12.90% | 12.03% |
| total amount of polysaccharides (%) | 0.15% | 0.07% | 0.07% | 0.19% |
| amount of fat (%) | 0.21% | 0.15% | 0.05% | 0.14% |
| microbial limits (CFU/g) | < 10 | < 10 | < 10 | < 100 |
| amount of endotoxin (EU/ ml) | < 0.02 | < 0.02 | < 0.02 | < 0.5 |
| retention rate of telopeptides (%) | 82.30% | 85.70% | 84.10% | / |
| N-terminal telopeptide | 83.60% | 87.50% | 85.60% | < 1ug/L |
| C-terminal telopeptide | 81.00% | 83.90% | 82.50% | < 2ug/L |

As shown in Table 2, compared to the commercial collagen, the collagen prepared by the method provided by the embodiments of the present disclosure has retention of the N-terminal telopeptide and the C-terminal telopeptide, the retention rate is more than 80%. The collagen of the present disclosure has high purity, the amount of collagen in the extract is more than 98%, and low contents of the protein impurities, total polysaccharides and fats, which are less than 0.5%, are contained. Low levels of microorganisms and endotoxins are contained. The amount of endotoxins is less than 0.02EU/ml. Therefore, the collagen of the present disclosure meets requirements of medical standards.

### 3. SDS-PAGE electrophoresis analysis

As shown in FIG. 6, bands of four samples are shown in the electrophoresis image. A first band is the marker of the electrophoresis. A second band, a third band and a fourth band are electrophoresis results of the collagen corresponding to the Embodiment 1, the Embodiment 2, and the Embodiment 3, respectively. As shown in FIG. 6, triple helix structures of the collagens of the Embodiment 1, the Embodiment 2, and the Embodiment 3 are unwound after electrophoresis. Molecular weights of an α1 chain and an α2 chain are approximately 100 Kda. Therefore, the results indicate that three helical chains of each collagen are retained. Various enzymes used in the preparation method do not significantly hydrolyze the triple helix structure. The peptide chains are intact and are not destroyed.

### 4. Circular dichroism (CD) analysis

The CD is used to characterize the triple-helical structure of the collagen. A CD of a physiological collagen has a negative peak at a wavelength of about 195 nm and has a positive peak at a wavelength of about 220 nm. The positive absorption peak is a typical feature of the circular dichroism of a L-configuration of polyproline. In addition to a position of the negative peak, it can be indicated that the triple-helical structure of the collagen is intact. If the positive peak at 220 nm is not detected, it is indicated that no triple helix structure exists. As a ratio of the positive peak to the negative peak is larger, the triple helix structure is more intact, and a content of the triple helix structure is higher.

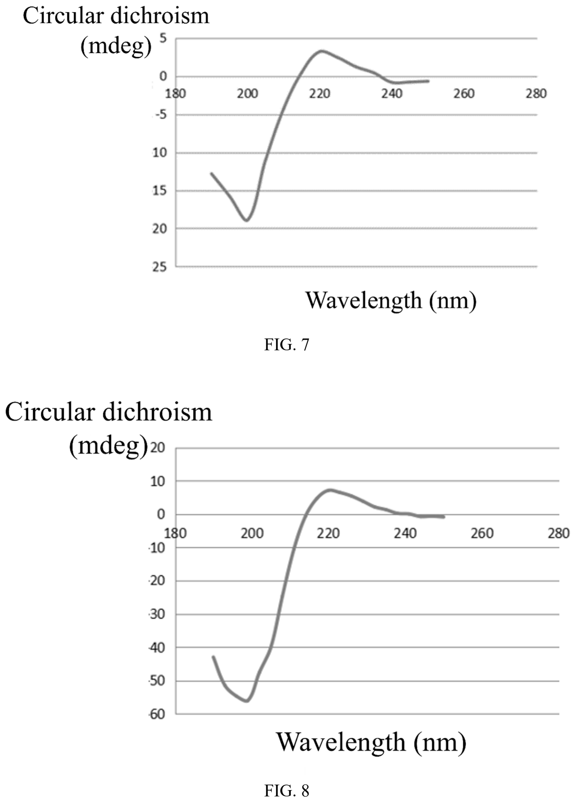

As shown in FIGS. 7 and 8, FIGS. 7 and 8 show circular dichroism results of the collagens corresponding to the Embodiment 1 and the Embodiment 2, respectively. The collagens of the Embodiment 1 and the Embodiment 2 each shows a characteristic positive peak at about 220 nm and shows a characteristic negative peak at about 195 nm. Therefore, the collagens of the Embodiment 1 and the Embodiment 2 each has the triple helical structure.

### 5. Differential calorimetric scanning (DSC) analysis

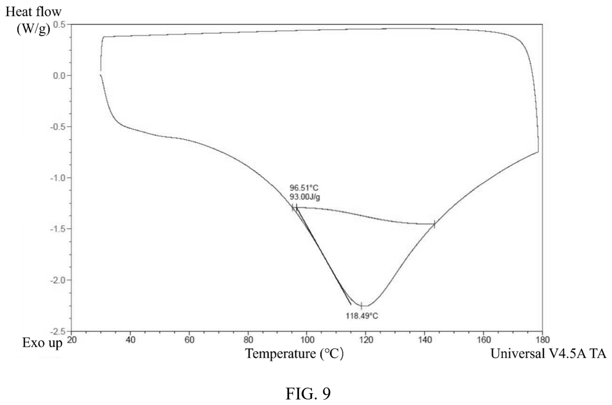

As shown in FIG. 9, FIG. 9 shows a differential calorimetric scanning pattern of the collagen corresponding to the Embodiment 3, and a melting peak at 118.49°C is shown, indicating that the collagen is structurally intact and has a high thermal melting temperature.

The above description describes only examples of the present disclosure, and does not limit the scope of the present disclosure. Any equivalent structure or equivalent process transformations performed based on contents of the specification and the accompanying drawings of the present disclosure, applied directly or indirectly in other related technical fields, shall all be equivalently included in the scope of the present disclosure.

## Claims

1. A method for preparing a telopeptide-retained collagen, comprising:
preparing a collagen suspension;
treating the collagen suspension with a protease to remove non-collagen protein impurities, so as to obtain a collagen extract, wherein the protease has a higher enzymatic efficiency for the non-collagen protein impurities than for a telopeptide of a collagen;
dissolving and homogenizing the collagen extract to obtain the telopeptide-retained collagen.

2. . The method according to claim 1, wherein, the treating the collagen suspension with a protease to remove non-collagen protein impurities, so as to obtain a collagen extract, comprises:
adding a neutral protease and/or an alkaline protease to the collagen suspension to obtain a first mixture, adjusting a pH of the first mixture to an optimal pH range in which the neutral protease and/or the alkaline protease having optimal activities;
leaving the first mixture to undergo reactions at a first predetermined temperature for a first predetermined time length to obtain a second mixture;
centrifuging, filtering and water washing the second mixture to obtain the collagen extract.

3. . The method according to claim 2, wherein, when the protease comprises the neutral protease, the optimum pH range for the neutral protease having the optimal activities is 6 to 8; and when the protease comprises the alkaline protease, the optimum pH range for the alkaline protease having the optimal activities is 7 to 10; and/or the first predetermined temperature is in a range of 4°C to 20°C; and/or, the first predetermined time length is in a range of 4h to 8h.

4. . The method according to claim 2, wherein, when adding the neutral protease and/or the alkaline protease to the collagen suspension, the method further comprises:
adding an enzyme stabilizer to the collagen suspension.

5. . The method according to claim 4, wherein the enzyme stabilizer comprises at least one of propylene glycol, polyethylene glycol, sucrose and mannitol.

6. . The method according to any one of claims 1 to 5, wherein, the protease comprises one or more of *Bacillus licheniformis* alkaline protease, *Aspergillus oryzae* alkaline protease, *Bacillus subtilis* alkaline protease, *Streptomyces griseus* alkaline protease and *Fusarium* alkaline protease.

7. . The method according to any one of claims 1 to 5, wherein, before treating the collagen suspension with the protease, the method further comprises:
adding a collagen terminal-group protector to the collagen suspension.

8. . The method according to claim 7, wherein, the collagen terminal-group protector comprises at least one of highly unsaturated fatty acids and amino acids.

9. . The method according to any one of claims 1 to 5, wherein, the dissolving and homogenizing the collagen extract to obtain the telopeptide-retained collagen, comprises:
adding an acidic solution to the collagen extract to obtain an acidic dissolving solution;
homogenizing the acidic dissolving solution under a high pressure to obtain the telopeptide-retained collagen.

10. . The method according to claim 9, wherein, the acidic solution comprises at least one of acetic acid, hydrochloric acid, sulfuric acid or citric acid.

11. . The method according to claim 9, wherein, the dissolving and homogenizing the collagen extract to obtain the telopeptide-retained collagen, comprises:
freezing and drying the telopeptide-retained collagen to obtain the telopeptide-retained collagen in a dried state.

12. . The method according to any one of claims 1 to 5, wherein, the preparing a collagen suspension, comprises:
selecting an animal tissue comprising the collagen;
refining the animal tissue into sub-tissues;
treating the sub-tissues sequentially with an organic reagent, a salt solution, an acid-enzyme mixture, and an alkaline solution, so as to obtain a pretreatment product; and
homogenizing and grinding the pretreatment product, so as to obtain the collagen suspension.

13. . The method according to claim 12, wherein, the homogenizing and grinding the pretreatment product, so as to obtain the collagen suspension, comprises:
transferring the pretreatment product to a homogenizing and grinding device, adding an alkaline solution, and homogenizing and grinding the pretreatment product to have a particle size of 20 µm to 400 µm, so as to obtain the collagen suspension.

14. . A telopeptide-retained collagen, prepared from the method according to any one of claims 1 to 13.

15. . The telopeptide-retained collagen according to claim 14, wherein, a telopeptide retention rate of the telopeptide-retained collagen is greater than or equal to 80%.
